# EUROPEAN PATENT APPLICATION

(11) **EP 2 541 249 A1**
(43) Date of publication of application: **02.01.2013**
(21) Application number: 11171657.7
(22) Date of filing: 28.06.2011
(51) Int. Cl.: G01N 33/543, G01N 33/574, G01N 33/68

(54) **EpCAM detection**

(71) Applicant: Oncotyrol Center for Personalized Cancer Medicine GmbH, 6020 Innsbruck (AT)
(72) Inventor: Spizzo, Gilbert, 39012 Merano (IT); Wurm, Martin, 6020 Innsbruck (AT)
(74) Representative: Sonn & Partner Patentanwälte

(57) **Abstract**

The invention discloses a method for detecting epithelial cell adhesion molecule (EpCAM) and/or the intracellular component of EpCAM (EpIC) and/or the extracellular region of EpCAM (EpEx) in a sample comprising the provision of a solid phase immunoassay wherein
- either a monoclonal antibody recognising EpEx or a monoclonal antibody recognising EpIC is immobilised as a first antibody on a solid surface;
- the first antibody is contacted with the sample to allow binding of EpCAM and/or EpIC and/or EpEx to the first antibody; and
- the EpCAM and/or EpIC and/or EpEx bound to the first antibody is contacted with a second antibody being either a monoclonal antibody recognising EpIC or a monoclonal antibody recognising EpEx to allow binding of EpCAM and/or EpIC and/or EpEx bound to the first antibody to the second antibody; and
- determining whether EpCAM and/or EpIC and/or EpEx have been recognised in the sample by the monoclonal antibodies.

## Description

The present invention relates to the determination of epithelial cell adhesion molecule (EpCAM) in a sample and kits for use in EpCAM detection.

EpCAM (also known as 17-1A, GA733-2, KSA, KS1/4, 323/A3; Seq ID No 1) is a calcium-independent homophilic intercellular cell adhesion molecule of 39-42 kDa which is expressed by the majority of epithelial tissues. The EpCAM antigen does not structurally resemble any of the major families of the adhesion molecules (cadherins, selectins, integrins, or cell adhesion molecules of the Ig superfamily). It is a type transmembrane glycoprotein, consisting of an extracellular domain with two EGF-like repeats, and a short cytoplasmic domain of 26 amino acids. The cDNA for EpCAM was cloned in 1989 from the lung adenocarcinoma cell line UCLA-P3 and designated KSA. The gene was mapped to human chromosome 2 by analysis of human-mouse somatic cell hybrids, by fluorescence in situ hybridisation and by PCR analysis (Spizzo et al., Cancer Lett. 246 (2007), 253-261; Spizzo et al., Br. Can. Res. Treat. 86 (2004), 207-213).

EpCAM shows frequent and high-level expression in numerous human malignancies including colon and breast cancers. EpCAM expression can increase with disease progression in prostate cancer and cervical intraepithelial neoplasia. High EpCAM expression is associated with poor disease-free and overall survival in invasive breast cancer. Silencing EpCAM gene expression with EpCAM short interfering RNA (siRNA) resulted in decreased cell proliferation of human breast cancer cell lines. Furthermore, EpCAM signalling seems to impact cell proliferation through up-regulation of the proto-oncogene c-myc. Beside its function as adhesion molecule, EpCAM can inhibit CD4⁺T-cell dependent immune responses and thus enable tumour cells to evade T-cell mediated antitumour immunity (Spizzo et al., 2007).

The EpCAM antigen has therefore attracted major interest as a target for passive and active immunotherapy. Antitumour responses have been observed in metastatic colorectal cancer after treatment with the EpCAM specific monoclonal antibody edrecolomab. Adjuvant treatment of radically resected Dukes' C colorectal cancer patients using the same antibody induced a 32% relative reduction in mortality as compared to the results of surgery alone. However, as compared to chemotherapy, treatment with the edrecolomab antibody in a phase III study was shown to be less effective. In vitro and in vivo analyses have shown that EpCAM expression can be up-regulated by chemotherapeutic agents increasing the effectiveness of EpCAM directed antibodies in terms of antibody-dependent cellular cytotoxicity. Thus, the EpCAM antigen has become an attractive target for antitumour immune interventions (Spizzo et al., 2007).

Due to its marker function (and also due to its proposed function as a tumour disease target) measurement of EpCAM in tumour patient samples is of importance for diagnosis, prediction and monitoring of therapy in cancer patients utilising either chemotherapeutics or specific monoclonal antibodies (or other tumour treatments or combinations thereof).

Determination of EpCAM in cancer patients, cell lines or other samples can be established by various methods including immunohistochemistry (Spizzo et al., 2004; Slanchev et al., PLoS Genetics 5 (2009), e1000563), ELISA (Abe et al., J. Immunol. Meth. 270 (2002), 227-233; Petsch et al., mAbs 3 (2011), 31-37), immunofluorescence, in-situ mRNA hybridisation (Gosens et al., Modern Path. 20 (2007), 221-232), immunoprecipitation (Maetzel et al., Nat. Cell Biol. 11 (2009), 162-171), flow cytometry, quantitative reverse transcriptase PCR (Q-RT-PCR) (Mitra et al., Mol. Vision 16 (2010), 828-842), etc.

Regulated intramembrane proteolysis activates EpCAM as a mitogenic signal transducer in vitro and in vivo. This involves shedding of its ectodomain EpEx and nuclear translocation of its intracellular domain EpIC. Cleavage of EpCAM is sequentially catalysed by TACE and presenilin-2. Pharmacological inhibition or genetic silencing of either protease impairs growth-promoting signalling by EpCAM, which is compensated for by EpIC. Released EpIC associates with FHL2, β-catenin and Lef-1 to form a nuclear complex ("EpCAM signalosome") that contacts DNA at Lef-1 consensus sites (thereby suggesting an association ("cross-talk") with the Wnt-pathway), induces gene transcription and is oncogenic in immunodeficient mice. In patients, EpIC was found in nuclei of colon carcinoma but not of normal tissue. Nuclear signalling of EpCAM explains how EpCAM functions in cell proliferation. The finding that EpCAM is a potent signal transducer that uses components of the Wnt pathway, with a proven significance for tumour development and cell renewal could explain the expression of EpCAM at high levels and with high frequency in cancer-initiating cells and their progeny, and normal stem or progenitor cells. For example, EpCAM is expressed in the mammalian germline and is frequently present at the surface of human and murine stem cells and cancer stem cells. Consequently, EpCAM-specific antibodies served in many studies to isolate not only cancer stem cells but also very rare circulating tumour cells from metastatic lung, prostate, pancreatic, breast and colon cancers. However, the regulation of EpCAM signalling in normal and malignant stem-type cells is as yet unexplored (Maetzel et al., 2009). Accordingly, there is a significant need to analyse and elucidate signals for EpCAM cleavage and regulation of proteases associated with its activation in normal and malignant stem cells. Such analyses are dependent on an unambiguous identification of the EpCAM species in a given sample. A clear and definite distinction and distinguishing between the total EpCAM (i.e. the whole EpCAM molecules comprising an EpIC and an EpEx moiety, e.g. soluble EpCAM (sEpCAM) present in e.g. serum of cancer patients ("serum EpCAM", Abe et al., 2002) but as well EpCAM derived from membranes), the EpEx and the EpIC is therefore needed. This need does not only exist in medicine but also in research.

It is an object of the present invention to provide improved means for EpCAM detection in samples, especially in samples of human tumour patients or patients being at risk of developing tumours or being suspected of having a tumour. Preferably, the present invention should provide means to specifically determine EpCAM, i.e. to discriminate between EpCAM and EpEx.

Therefore, the present invention provides a method for detecting epithelial cell adhesion molecule (EpCAM) and/or the intracellular component of EpCAM (EpIC) and/or the extracellular region of EpCAM (EpEx) in a sample comprising the provision of a solid phase immunoassay wherein
- either a monoclonal antibody recognising EpEx or a monoclonal antibody recognising EpIC is immobilised as a first antibody on a solid surface;
- the first antibody is contacted with the sample to allow binding of EpCAM and/or EpIC and/or EpEx to the first antibody; and
- the EpCAM and/or EpIC and/or EpEx bound to the first antibody is contacted with a second antibody being either a monoclonal antibody recognising EpIC or a monoclonal antibody recognising EpEx to allow binding of EpCAM and/or EpIC and/or EpEx bound to the first antibody to the second antibody; and
- determining whether EpCAM and/or EpIC and/or EpEx have been recognised in the sample by the monoclonal antibodies.

Specifically preferred monoclonals applied in the present invention recognise amino acids nos. 305 to 314 (Seq ID No 2) of the human EpCAM protein (as EpIC antibody) or amino acids nos. 75 to 88 (Seq ID No 3) of the human EpCAM protein (as EpEx antibody). The amino acid numbering refers to the amino acid numbering of the 314 amino acid long human EpCAM protein deposited in the UniProtKB/Swiss-Prot database under the Accession number P16422 (EPCAM_HUMAN; Seq ID No 1). "recognizing" the amino acids nos. 305 to 314 or amino acids no. 75 to 88 of the human EpCAM protein means that the antibody specifically binds to this region. Amino acids no. 305 to 314 is the most C-terminal portion of the EpCAM protein and also the most C-terminal part of the EpIC fragment of EpCAM. The N-terminal Cys residue in Seq ID Nos 2 and 3 is preferably present (at the C- or N-terminus; preferably at the N-terminus (especially in Seq ID No 2) in order to bind the EpCAM peptides to carriers or determine antibody binding specificities .

The present invention provides a highly specific determination of EpCAM and allows a clear and unambiguous discrimination between EpCAM and EpIC and EpEx. Since EpCAM can be cleaved in the organism to the EpIC and the EpEx fragment, binding of a molecule to an anti-EpCAM antibody which is specific for an epitope in the EpEx fragment cannot unequivocally indicate that EpCAM is the molecule bound by the antibody; it could be EpEx as well. The same holds true for the binding to an antibody which is specific for an epitope on the EpIC fragment. Binding could indicate EpCAM as well as EpIC. Accordingly, none of the ELISA systems according to the prior art is capable of EpCAM specific determination, because none of these tests can distinguish between EpCAM and the cleavage product EpEx, because EpEx and EpCAM are two different proteins.

For example, Abe et al., 2002, disclose an ELISA where a monoclonal antibody against EpEx (EpEx was termed "sMK-1" in Abe et al.) is immobilised in microtiter plate wells (the immobilised antibody was termed "M2-5"). EpEx is then bound to the immobilised antibody. Bound EpEx is then incubated with another monoclonal antibody against EpEx (termed "M4-10") which recognises a different epitope than the immobilised antibody. It is clear that this ELISA cannot discriminate between EpEx and EpCAM. In a similar manner, however, even with a polyclonal instead of a monoclonal antibody, Petsch et al., 2011, provide an electroluminescence-based ELISA for quantification of EpCAM. A biotinylated polyclonal goat-anti-human antibody which was raised against a mouse myeloma cell line NSO derived recombinant human EpEx domain (Gln24-Lys265 of EpCAM) was bound to a streptavidin-coated microplate. Then serum samples or serially diluted recombinant EpEx spiked in human serum were bound to the immobilised polyclonal goat-anti-human antibody. Then, a monoclonal mouse anti-human EpCAM antibody (termed "5-10" of Micromet AG, Munich, Germany was bound to the captured recombinant EpEx and detected via a goat anti-mouse antibody conjugated to SulfoTag^{™} (MSD, R32AC-5). Again, discrimination between EpEx and whole EpCAM is not possible with this assay.

In Maetzel et al., 2009, FaDu cells were stained with a monoclonal EpEx-antibody (HO-3; Ruf et al., Br. J. Cancer 97 (2007), 315-321) and polyclonal EpIC-specific antibodies (affinity purified goat polyclonal antibody raised against a peptide mapping at the C-terminus of human EpCAM) in combination with Alexa-488 and Alexa-647 secondary antibodies, respectively. Localization of EpEx (green) and EpIC (red) was assessed with laser scanning microscopy.

With the present invention, a reliable, robust and very specific method for determining EpCAM is provided, which is specifically suited for routine testing. It allows determining EpCAM presence and concentrations in samples, especially samples of human cancer patients or humans being suspected or having a risk of having cancer, in an efficient manner. With the present invention, any form of EpCAM can be detected and specifically identified. Especially whole EpCAM, i.e. the EpCAM molecule having an EpEx as well as an EpIC portion, can be identified and - in one and the same test - distinguished from EpCAM fragments, such as the EpEx and the EpIC being present in the same sample.

With this solid phase immunoassay, a reliable and robust technology is provided which allows immediate and precise answers as to which of the EpCAM species (EpCAM, EpEx, EpIC) is present in a sample without the need to perform a size separation (such as gel electrophoresis) which bears the risk of unreliable results due to denaturing processes. For example, if the EpEx recognising antibody is bound to the solid surface ("first antibody"; since it is preferred to immobilise the first antibody to the solid surface before contact to the sample or the second antibody, it may preferably also be referred to as the "first immobilised antibody"), binding of the EpIC antibody ("second antibody") proves the presence of EpCAM in the sample. Binding of an EpCAM species to the immobilised antibody without binding of the second antibody proves the presence of EpEx in the sample. On the other hand, if the EpIC antibody is bound to the solid surface, binding of the EpEx antibody shows again the presence of EpCAM in the sample whereas binding of an EpCAM species to the immobilised antibody without detection by the second antibody against EpEx shows presence of EpIC in the sample.

Determination of the binding events (only EpCAM species binds to immobilised antibody; second antibody binds to EpCAM species bound by the immobilised first antibody) can be performed by various methods established for ELISA/immunosorbent assays. For example, binding of the EpCAM species (without binding of the second antibody) can be determined by a reagent identifying the binding event between the immobilised antibody and the EpCAM species (e.g. an antibody specifically recognising the immune complex between the immobilised immunoglobulin and the EpCAM species ("specifically" meaning that neither the (empty) immobilised antibody nor the non-bound EpCAM species is recognised by this antibody).

The method according to the present invention is specifically suited to detect soluble EpCAM (sEpCAM), especially sEpCAM in the cytosol of isolated cancer cells or EpCAM in blood, plasma or serum samples isolated from humans. This sEpCAM species has surprisingly turned out to be a very meaningful indicator for poor prognosis of cancer patients in the course of the present invention. The role of EpCAM in cancer has recently been reclassified. Historically, EpCAM is a tumour antigen and the first one defined by antibody. In breast cancer and other solid tumours it has been shown by many groups that EpCAM is over-expressed up to 1000fold. Overexpression of EpCAM is correlated with poor prognosis in breast cancer patients.

Recently, it was reported that EpCAM has oncogenic features in FaDu cells upon proteolytic cleavage into an extracellular part EpEx and a short intracellular domain EpIC. Although these results do not seem to be directly applicable to the situation in breast cancer cell lines or primary breast epithelia, it could be established in the course of the present invention that in four commercially available EpCAM over-expressing cell lines massive amounts of highly glycosylated protein is present in the cytosol. Upon cell lysis, full length glycosylated EpCAM but neither of the two EpCAM cleavage products EpEx or EpIC is found in the cytosolic lysate, proven by Western blot analysis. Also, when cells were lysed in RIPA buffer to solubilise membrane bound EpCAM; neither cleavage product could be identified.

Based on these findings, it was therefore necessary to develop a reliable immunosorbent test, especially in a suitable ELISA system format, for the monitoring of EpCAM positive tumour cell lysis, initially based on EpIC analysis in tumour patient samples. The finding that on the one hand there is no EpCAM proteolysis in breast cancer cell lines but on the other hand there is a massive amount of glycosylated EpCAM in the cytosol of these cells provided the target, EpCAM.

More specifically, the present invention identified for the first time EpCAM as a monitoring target for specific tumour cell lysis in cancer patients with EpCAM over-expressing phenotype, especially in breast cancer patients. In view of these findings, the need for establishing the test according to the present invention is even more evident.

Preferably, either the monoclonal antibody recognising EpIC or the monoclonal antibody recognising EpEx or both is labelled. The label is preferably an enzyme (as in the classical" ELISA), a fluorophore, a chemiluminescent material, a radioisotope, or a coenzyme. Generally enzyme labels such as alkaline phosphatase or beta galactosidase are employed together with their appropriate substrates. The enzyme/substrate reaction can be detected by any suitable means such as spectrophotometry.

According to a preferred embodiment, the determination whether EpCAM and/or EpIC and/or EpEx have been recognised in the sample by the monoclonal antibodies is performed by using secondary antibodies binding to the monoclonal antibodies, said secondary antibodies being preferably labelled, especially with a fluorescence label, a luminescence label, a colourigenic label; a radioactive label, a biotin or biotin analogue label, or combinations thereof.

The EpCAM species bound to the immobilised mAb or the (second) antibody bound to the EpCAM species can, in turn, be detected by having the antibody labelled directly or indirectly with a detectable marker. Alternatively, the EpIC/EpEx-specific antibody can be contacted with a secondary antibody which is specific for the EpIC/EpEx-specific antibody. This secondary antibody can be labelled directly or indirectly with a detectable marker.

Another preferred embodiment relates to a method wherein the monoclonal antibody recognising the EpIC and the monoclonal antibody recognising EpEx are labelled with different labels, preferably with different fluorescence labels. Also quenching techniques can be applied. Moreover, also the EpCAM species in the sample can be labelled. It is also possible to include competitive techniques, e.g. by the addition of known amounts of an EpCAM species competing with the EpCAM species in the sample. For example, a labelled EpCAM can be added (e.g. a fluorescent or quencher labelled EpCAM).

According to another embodiment, the solid surface contains a capturing molecule for one of the mAbs (either the EpIC or the EpEx mAb), especially an antibody binding the EpEx or the EpIC mAb according to the present invention (e.g. a secondary antibody as disclosed above). Both mAbs according to the present invention (EpEx and EpIC) can be brought into the sample to allow binding to EpCAM, EpEx or EpIC. EpIC or EpEx are then bound to the surface via the capturing molecule binding the mAb recognising EpEx or EpIC; if the bound species also carries the second antibody, presence of EpCAM is detected according to the present invention. Presence of both mAbs according to the present invention in a form bound to the surface can be detected by various means, e.g. by different labels on the two mAbs according to the present invention or by interaction of the labels (e.g. a quenching or non-quencing effect if both mAbs are present in close distance (which is the case if both are present in a form bound to the surface captured by the capturing molecule.

The method according to the present invention can be performed in various practical embodiments known in the present field of surface based immunoassays. For example, the method can be provided as an enzyme linked immunosorbent assay (ELISA).

For providing the method according to the present invention, it is essential to provide the antibodies used in a precisely defined manner, i.e. as a single molecular species. This is highlighted by the requirement of a "monoclonal antibody" ("mAb"). This term includes all forms of antibody molecules being derivable (synthetically or biologically) from a monoclonal antibody, such as the whole mAb or only parts thereof (Fab, Fab' and F(ab')₂, Fd, single-chain Fvs (scFv), single-chain immunoglobulins (e.g., wherein a heavy chain, or portion thereof, and a light chain, or portion thereof, are fused), disulfidelinked Fvs (sdFv), diabodies, triabodies, tetrabodies, scFv minibodies, Fab minibodies, and dimeric scFv and any other fragments comprising a V_{L} and a V_{H} domain in a conformation such that a specific complementarity determining region (CDRs) is formed (or only the CDR provided in a suitable scaffold which still allows binding of the molecule to the EpCAM species (either the EpIC or EpEx epitope)). Antigen-specific fragments may also comprise a V_{H}H domain derived from a camelid antibody. The V_{H}H may be engineered to include CDRs from other species, for example, from human antibodies. Alternatively, a human-derived heavy chain V_{R} fragment may be engineered to resemble a single-chain camelid CDR, a process referred to as "camelization". However, in contrast to therapeutic applications of antibodies, the very nature of the antibodies used according to the present invention is mainly driven by the suitability for the detecting purposes according to the present invention. The major prerequisites are the binding specificities to the EpCAM species and the detectability of the binding event (e.g. by labelling, affinity to further binding molecules, such as antibodies, etc.). Mimetics of the antibodies can also be used in the present invention as long as these mimetics are defined with respect to their molecular structure. Accordingly, the term "monoclonal antibody" mainly serves to exclude EpCAM binding preparations of inhomogeneous or undetermined binding nature, such as polyclonal antibodies.

With the method according to the present invention, any sample which potentially contains EpCAM, EpEx or EpIC can be analysed and determined. Preferred samples are, of course, samples provided in connection with tumour diagnostics and tumour therapy. Therefore, the present method is preferably performed in samples of human tumour patients or patients being at risk of developing tumours or being suspected of having a tumour. The present method is especially suitable for monitoring tumour patients during therapy since EpCAM/EpEx/EpIC could be important markers for development of the tumour disease and the effectiveness of tumour therapy. According to a preferred embodiment of the present invention the sample is ascites, pleural effusions, blood or blood derived samples, such as serum or plasma; a tumour cell lysate, tumour tissue, or urine.

The major target tumour diseases are those for which EpCAM is known to be a tumour marker, i.e. expressed in the tumour cells of the patient. The present invention is specifically suited for tumour diseases where presence and/or increase of EpCAM expression is known to correlate with poor prognosis. Accordingly, the method according to the present invention is preferably applied in diagnosing and monitoring samples from tumour patients having or being suspected to have (or being at risk to have or develop) colon cancer, breast cancer, prostate cancer and cervical cancer, especially a cervical intraepithelial neoplasia.

Attachment of the mAb to a solid support can be performed according to conventional methods used in the art. The term "solid support" or "solid surface" refers to a material having a solid surface to which a mAb according to the present invention is immobilised. By "immobilised" it is meant bound covalently, or bound by noncovalent means such as hydrophobic adsorption. A solid support may be the surface of a multiwell (microtiter) plate well, a bead, a membrane or a dipstick. Methods and means for covalently or noncovalently binding proteins to solid supports are known in the art. Suitable solid supports include latex, glass particles, including porous glass particles; polyacrylamide particles; agarose; Sephadex^{®} (Pharmacia Fine Chemicals, Inc.); Sepharose^{®}; bibulous materials such as glass or cellulose paper; plastics and polymers (e.g., in sheets, beads or microtiter wells) such as polystyrene, polyvinyl chloride, polystyrene latex, or polyvinylidine fluoride (known as Immulon^{®}); nylon; polymethacrylate; etc.; silicons; metals such as gold and indium; nitrocellulose (e.g., in membrane or microtiter well form); activated beads; Protein A beads; diazotised paper; and the like. The nature of the solid surface varies depending upon the specifically intended use or method. For assays carried out in microtiter wells, e.g., in multiwell (microtiter) plates, the solid surface is the wall of the well or cup. For assays using beads, the solid surface is the surface of the bead. In assays using a dipstick (i.e., a solid body made from a porous or fibrous material such as fabric or paper) the surface is the surface of the material from which the dipstick is made. In agglutination assays the solid surface may be the surface of latex or gelatin particles. When individual antigens are bound to a solid surface they may be distributed homogeneously on the surface or distributed thereon in a pattern, such as bands so that a pattern of antigen binding may be discerned. The antibodies can also be bound to a surface which specifically recognises the binding of a ligand (EpCAM, EpEx or EpIC) to the immobilised antibody. Biochips can be designed to detect the binding of the ligand and would therefore allow to discriminate between a bound ligand which is recognised by the second mAb according to the present invention (EpCAM) and a bound ligand which is not recognised by the second mAb (EpIC or EpEx) allowing the immediate and simultaneous detection of EpIC and EpEx in one and the same set-up (EpEx or EpIC would result in a binding signal only whereas EpCAM results in binding and detection by the second antibody). Such techniques are e.g. surface plasmon resonance detection, atomic force detection, electrochemical impedance spectroscopy, silicon nanowires, functionalised carbon nanotube biosensors, centrifugal microfluidic and bio-optical disks, etc. (see e.g. Nolte, Rev. Sci. Instr. 80 (2009), 101101; McNeil, J. Leuk. Biol. 78 (2005), 585-594; Özkumur et al., PNAS 105 (2008), 7988-7992; Lin et al., PNAS 107 (2010), 1047-1052; Lausted et al., Mol. Cell. Prot 7 (2008), 2464-2474; all with further references).

According to another aspect, the present invention provides a kit for detecting EpCAM and/or EpEx and/or EpIC in a sample comprising:
- a surface for binding monoclonal antibodies and performing the immunological testing according to the present invention,
- a monoclonal antibody recognising EpIC; and
- a monoclonal antibody recognising EpEx.

Preferably, the kit according to the present invention further comprises detection agents for detecting the monoclonal antibodies and/or detection means for detection of a binding event between the antibody and EpCAM in a sample. Preferably, the respective detection antibody is marked for specific detection, e.g. biotinylated.

In the solid phase assay according to the present invention, either the monoclonal antibody recognising EpIC or the monoclonal antibody recognising EpEx is immobilised on a solid surface. According to a preferred embodiment, the kit according to the present invention therefore already contains one of the mAbs immobilised to the solid surface, especially the mAb recognising EpEx immobilised to the solid surface (in this embodiment, the EpIC antibody is provided in soluble form or in a form which can be brought into such soluble form (e.g. lyophilised or frozen)). This allows an improved serial testing of the samples, especially in serial testing formats due to the robustness of the detection method.

Preferably, the kit according to the present invention further comprises an EpCAM standard, preferably a recombinant EpCAM protein, a recombinant EpIC protein, a recombinant EpEx protein, or combinations thereof.

Preferably, the kit according to the present invention further comprises a standard, e.g. a defined EpCAM and/or EpIC and/or EpEx standard (preferably all three standards), a capture antibody, a biotinylated detection antibody, e.g. a suitable biotinylated (or otherwise labelled (chromogenic, luminescent, colourigenic, fluorescent, etc.)) antibody against the monoclonal EpIC and/or EpIC antibody, containers for the sample(s) (e.g. an ELISA plate), use instructions, written standard information, especially tables of standard values of EpCAM levels in healthy humans or cancer patients.

In a preferred embodiment, the present invention applies more than one mAb for recognising EpIC and/or EpEx. It is specifically preferred to provide a test wherein a second monoclonal antibody for EpEx is provided which does not interfere with the binding of the first monoclonal antibody recognising EpEx ("does not interfere" means that these antibodies do not compete with each other (at the same binding site)). For example, the preferred monoclonal antibody against EpEx (binding to aa 75 to 88 of EpCAM) does not compete with catumaxomab; accordingly, catumaxomab may be combined with the test kit and method of the present invention. For example, the second EpEx antibody may be used to confirm presence of EpEx in an immunosorbent assay based on an immobilised EpEx mAb. Non-binding of the EpIC mAb in the test according to the present invention confirms absence of EpCAM in the sample; additional testing with the second EpEx mAb can confirm presence or absence of EpEx in the same test (especially, if the second mAb for EpEx has a different label or can be differently detected than the EpIC antibody (e.g. two different fluorescence labels). The same design can, of course apply if the EpIC antibody is immobilised, although the possibilities for providing a second EpIC antibody not competing with the first one are restricted and therefore less attractive for routine testing, specifically in view of the large number of EpEx mAbs already available.

Accordingly, a specific aspect of the present invention also deals with the monitoring of a tumour treatment which is based on an anti-EpCAM antibody, such as catumaxomab, edrecolomab, adecatumumab, oportuzumab monatox, tucotuzumab celmoleukin and citatuzumab bogatox. The method and kit of the present invention are specifically suitable for such monitoring.

The invention is further illustrated by the following examples and the drawing figures, yet without being restricted thereto.
Fig. 1 shows Western blot analysis of breast cancer cell lines overexpressing EpCAM: Lysates of BT-20 (1), MCF-7 (2), SkBr-3 (3), T47D (4) cells have been detected with 9-2 (A) and 54-7 EpCAM antibodies (B); Tubulin has been used as loading control;
Fig. 2 shows comparison of different antibodies: mAb 9-2 detects whole EpCAM in lysates of MCF-7 cells in WB; signal is similar to that of an EpIC polyclonal antibody (A-20) and of an antibody targeting the extracellular domain of EpCAM (C-10, Santa Cruz); and
Fig. 3 shows IHC of EpCAM overexpressing colon cancer tissue: A: stain with 9-2 mAb. B: stain with NCL-ESA mAb (Novocastra).

### EXAMPLES

### Materials and Methods

### Monoclonal Antibodies

Two monoclonal mouse antibodies (mAb) were generated against epitopes in the extra- and intracellular domain of human EpCAM, respectively. BALB/c mice (female, approx 8 weeks) from Janvier (France) were used to generate hybridomas. For fusion, SP2/0-Ag14 cells (Deutsche Sammlung von Mikroorganismen und Zellkulturen) were used. After immunisation and test of serum, isolated thymocytes were fused with SP2/0 cells. Positive clones were further subcloned. Immunisation, fusion and subcloning was performed by Biogenes GmbH, Berlin. The first antibody 9-2 recognises the C-terminus of the intracellular domain of human EpCAM with the sequence C-MGEMHRELNA (Seq ID No 2). The second antibody 54-7 is directed against the sequence C-GSKLGRRAKPEGAL (Seq ID No 3) located in the extracellular region of EpCAM. Peptides for immunisation were produced by Biosyntan GmbH, Berlin.

Hybridoma were grown in ISF-1 medium (Biochrom AG, Berlin) and supernatants were harvested and sterile filtered. Antibodies were isolated from the supernatant by protein G chromatography and subsequently concentrated and rebuffered in phosphate buffered saline at pH 7, sterile filtered and aliquots thereof were stored at -35°C. Purity of mAbs was controlled by SDS-PAGE and concentration of mAbs was determined with a ND-1000 spectrophotometer (Peqlab Biotechnologie GmbH, Erlangen).

mAb 9-2 was biotinylated using sulfo-NHS-LC-Biotin from Thermo Scientific according to the manufacturer's instructions.

### EpCAM standard

Lysates of MCF-7 and Skbr-3 cells (ATCC) were pooled and aliquots thereof were stored at -35°C. The concentration of EpCAM in aliquots was calculated by comparing with a recombinant EpCAM standard purchased from Sino Biological Inc. (Beijing).

For calculation of background, a pool of 15 pre-tested control sera was used as negative control.

### ELISA

The ELISA for the detection of soluble human EpCAM (sEpCAM) in serum and ascites of cancer patients was performed using a sandwich ELISA system in standard 96well microtiter plates (BD Bioscience). For coating, 54-7 mAb directed against the extracellular EpCAM domain was used at a concentration of 0.5 µg/ml in PBS pH 7.0 at 4°C overnight. After washing with PBS 0.05% Tween 20 wells were blocked with PBS 0.1% BSA for 20 min at RT. EpCAM standard and samples were diluted in sample buffer consisting of PBS 0.1% BSA 0.05% Tween 20 in serial dilution and incubated for 1h at RT. After washing, the biotinylated detection antibody 9-2-bio was added at a concentration of 1 µg/ml in sample buffer for 1h at RT. After washing, streptavidinperoxidase ultrasensitive (Sigma) was added 1:10.000 in PBS 0.5% BSA. TMB (Southern Biotech) was used as substrate. Colour development was detected at 450/655 nm using a benchmark microplate reader (Biorad).

Sera and ascites were categorised into four groups according to their results in EpCAM specific ELISA: negative (-) signal < 3x background; slightly positive (+) signal 3-5x background; positive (++) signal 5-7x background; highly positive (+++) signal > 7x background signal.

### Human serum and ascites samples

Patients and control donors who donated blood or ascites samples signed an informed consent form.

### Cell lines and culture

BT-20, MCF-7, Skbr-3 and T47-D cells were purchased from the American type cell culture collection (ATCC). BT-20 and MCF-7 cells were grown in MEM Eagle medium (PAN Biotech, Passau) supplemented with 10% FCS (PAA, Linz), 2mM glutamine, 100 U/ml of penicillin and 100µg/ml of streptomycin (P/S, all from Invitrogen, Carlsbad, CA). Skbr-3 were grown in McCoy's 5A medium (PAN Biotech) supplemented with 10% FCS, 2mM glutamine and P/S. T47-D cells were grown in RPMI 1640 medium (PAN Biotech) plus 10% FCS, 2mM glutamine and P/S. For the generation of lysates, cells were harvested in RIPA buffer (Sigma-Aldrich, St. Louis, MO).

### Western Blot

For immunoblot, cells were harvested in RIPA buffer, transferred onto nitrocellulose membrane (Millipore, Billerica, MA) and detected using 9-2 and 54-7 antibody in combination with horseradish (HRP)-conjugated polyclonal anti-mouse antibody and the enhanced chemiluminescence (ECL) system (Amersham Bioscience/GE Healthcare, Uppsala).

### Immunohistochemistry

Immunohistochemistry (IHC) of tumour samples was performed according to Spizzo et al. (J. Clin. Pathol. 64, 415-420 (2011)).

### Results

### ELISA of human sera and ascites

As shown in table 1, a total of 29 healthy control sera were tested in sEpCAM ELISA. One sample was slightly positive (+), the rest was negative for sEpCAM. Furthermore, a total of 65 tumour sera were analysed (table 1) for the presence of sEpCAM. Nearly two thirds of the tested sera were negative. One fifth of the positive sera were slightly positive (+), one eighth were positive (++) and three cases were highly positive (+++) for sEpCAM. 15 of the negative sera were pooled for determination of background signal.

In ascites, the distribution of negative and positive cases was very similar to that in tumour sera. It was found that out of 23 tested ascites roughly two thirds were negative for sEpCAM and among the positive samples there were twice as many slightly positive (+) ones than positive (++) ones and no highly positive (+++) at all (table 1).

**Table 1: sEpCAM ELISA of human control and tumour sera and of ascites. Values are %. Negative cases (neg), the sum of positive cases ∑(+) and differently high sEpCAM samples (+, ++, +++) are shown.**

| | neg | ∑ (+) | pos (+) | pos (++) | pos (+++) |
|---|---|---|---|---|---|
| control (n=29) | 96,6 | 3,4 | 3,4 | 0, 0 | 0, 0 |
| tumor (n=65) | 61,5 | 38,5 | 21,5 | 12,3 | 4,6 |
| ascites (n=23) | 60,9 | 39,1 | 28,3 | 10,9 | 0,0 |

### Western blot of EpCAM overexpressing tumour cell lines

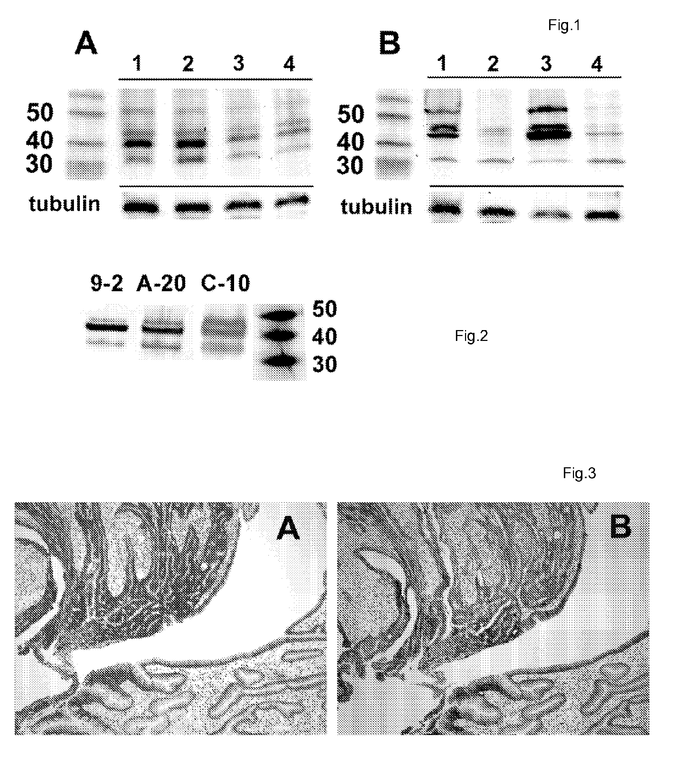

As shown in Figure 1, antibodies 9-2 and 54-7 are able to detect EpCAM in lysates of EpCAM overexpressing tumour cell lines. EpCAM has a MW of around 34 kD (non-glycosylated) and of around 41 kD (glycosylated). In tumour cell lines, EpCAM is usually present in highly glycosylated versions. In Figure 2, EpCAM in lysates of MCF-7 cells is depicted using commercially available EpCAM antibodies in comparison with the 9-2 mAb.

### Immunohistochemistry of EpCAM overexpressing tissue

In Figure 3, IHC of EpCAM overexpressing tissue is depicted using the commercial standard EpCAM antibody NCL-ESA in comparison with the 9-2 mAb. Figure 3 depicts a section of colon tissue. Cell nuclei (blue colour) are stained with haematoxylin. EpCAM (brown colour) is stained using the commercial standard NCL-ESA (A) or mAb 9-2 (B) in combination with horseradish peroxidase. These results show that mAb 9-2 is a suitable alternative for NCL-ESA in immunohistochemistry.

## Claims

1. Method for detecting epithelial cell adhesion molecule (EpCAM) and/or the intracellular component of EpCAM (EpIC) and/or the extracellular region of EpCAM (EpEx) in a sample comprising the provision of a solid phase immunoassay wherein
- either a monoclonal antibody recognising EpEx or a monoclonal antibody recognising EpIC is immobilised as a first antibody on a solid surface;
- the first antibody is contacted with the sample to allow binding of EpCAM and/or EpIC and/or EpEx to the first antibody; and
- the EpCAM and/or EpIC and/or EpEx bound to the first antibody is contacted with a second antibody being either a monoclonal antibody recognising EpIC or a monoclonal antibody recognising EpEx to allow binding of EpCAM and/or EpIC and/or EpEx bound to the first antibody to the second antibody; and
- determining whether EpCAM and/or EpIC and/or EpEx have been recognised in the sample by the monoclonal antibodies.

2. Method according to claim 1, wherein the monoclonal antibody recognising EpIC binds to amino acids no. 305 to 314 of the human EpCAM protein.

3. Method according to claim 1 or 2, wherein the monoclonal antibody recognising EpEx binds to amino acids no. 75 to 88 of the human EpCAM protein.

4. Method according to any one of claims 1 to 3, wherein the method is used to detect soluble EpCAM (sEpCAM), especially sEpCAM in the cytosol of isolated cancer cells or EpCAM in blood, plasma or serum samples isolated from humans.

5. Method according to any one of claims 1 to 4, wherein either the monoclonal antibody recognising EpIC or the monoclonal antibody recognising EpEx or both is labelled.

6. Method according to any one of claims 1 to 4, wherein determining whether EpCAM and/or EpIC and/or EpEx have been recognised in the sample by the monoclonal antibodies is performed by using secondary antibodies binding to the monoclonal antibodies, said secondary antibodies being preferably labelled, especially with a fluorescence label, a luminescence label, a colourigenic label; a radioactive label, a biotin label, or combinations thereof.

7. Method according to any one of claims 1 to 6, wherein the monoclonal antibody recognising EpIC and the monoclonal antibody recognising EpEx are labelled with different labels, preferably with different fluorescence labels.

8. Method according to any one of claims 1 to 7, wherein the method is an enzyme linked immunosorbent assay (ELISA), an immunocytochemistry assay, a detection of circulating tumour cells or tumour stem cells assay, or a detection of circulating cells or stem cells assay.

9. Method according to any one of claims 1 to 8, wherein the sample is ascites, pleural effusions, blood or a blood derived sample, especially serum or plasma; a tumour cell lysate, tumour tissue, or urine.

10. Method according to any one of claims 1 to 9, wherein the sample is a sample of a breast, colon, prostate or cervical cancer patient.

11. Kit for detecting EpCAM and/or EpIC in a sample comprising:
- a solid surface for binding monoclonal antibodies,
- a monoclonal antibody recognising EpIC; and
- a monoclonal antibody recognising EpEx.

12. Kit according to claim 11, further comprising detection agents for detecting the monoclonal antibodies and/or detection means for detection of a binding event between the antibody and EpCAM in a sample.

13. Kit according to claim 11 or 12, wherein either the monoclonal antibody recognising EpIC or the monoclonal antibody recognising EpEx is present in immobilised form on the solid surface.

14. Kit according to any one of claims 11 to 13, wherein it further comprises an EpCAM standard, preferably a recombinant EpCAM protein, a recombinant EpIC protein, a recombinant EpEx protein, or combinations thereof.

15. Kit according to any one of claims 11 to 14, wherein the solid surface comprises the monoclonal antibody recognising EpEx in immobilised form and wherein the monoclonal antibody recognising EpIC is present in soluble form or in a form which can be brought into soluble form.


SEQUENCE LISTING

<110>  ONCOTYROL - Center for Personalized Cancer Medicine GmbH

<120>  Detection of EpCAM

<130>  R 59071

<160>  3

<170>  PatentIn version 3.5

<210>  1
<211>  314
<212>  PRT
<213>  homo sapiens

<400>  1

Met Ala Pro Pro Gln Val Leu Ala Phe Gly Leu Leu Leu Ala Ala Ala
1               5                   10                  15

Thr Ala Thr Phe Ala Ala Ala Gln Glu Glu Cys Val Cys Glu Asn Tyr
            20                  25                  30

Lys Leu Ala Val Asn Cys Phe Val Asn Asn Asn Arg Gln Cys Gln Cys
        35                  40                  45

Thr Ser Val Gly Ala Gln Asn Thr Val Ile Cys Ser Lys Leu Ala Ala
    50                  55                  60

Lys Cys Leu Val Met Lys Ala Glu Met Asn Gly Ser Lys Leu Gly Arg
65                  70                  75                  80

Arg Ala Lys Pro Glu Gly Ala Leu Gln Asn Asn Asp Gly Leu Tyr Asp
                85                  90                  95

Pro Asp Cys Asp Glu Ser Gly Leu Phe Lys Ala Lys Gln Cys Asn Gly
            100                 105                 110

Thr Ser Met Cys Trp Cys Val Asn Thr Ala Gly Val Arg Arg Thr Asp
        115                 120                 125

Lys Asp Thr Glu Ile Thr Cys Ser Glu Arg Val Arg Thr Tyr Trp Ile
    130                 135                 140

Ile Ile Glu Leu Lys His Lys Ala Arg Glu Lys Pro Tyr Asp Ser Lys
145                 150                 155                 160

Ser Leu Arg Thr Ala Leu Gln Lys Glu Ile Thr Thr Arg Tyr Gln Leu
                165                 170                 175

Asp Pro Lys Phe Ile Thr Ser Ile Leu Tyr Glu Asn Asn Val Ile Thr
            180                 185                 190

Ile Asp Leu Val Gln Asn Ser Ser Gln Lys Thr Gln Asn Asp Val Asp
        195                 200                 205

Ile Ala Asp Val Ala Tyr Tyr Phe Glu Lys Asp Val Lys Gly Glu Ser
    210                 215                 220

Leu Phe His Ser Lys Lys Met Asp Leu Thr Val Asn Gly Glu Gln Leu
225                 230                 235                 240

Asp Leu Asp Pro Gly Gln Thr Leu Ile Tyr Tyr Val Asp Glu Lys Ala
                245                 250                 255

Pro Glu Phe Ser Met Gln Gly Leu Lys Ala Gly Val Ile Ala Val Ile
            260                 265                 270

Val Val Val Val Ile Ala Val Val Ala Gly Ile Val Val Leu Val Ile
        275                 280                 285

Ser Arg Lys Lys Arg Met Ala Lys Tyr Glu Lys Ala Glu Ile Lys Glu
    290                 295                 300

Met Gly Glu Met His Arg Glu Leu Asn Ala
305                 310

<210>  2
<211>  11
<212>  PRT
<213>  artificial

<220>
<223>  EpCAM peptide

<400>  2

Cys Met Gly Glu Met His Arg Glu Leu Asn Ala
1               5                   10

<210>  3
<211>  15
<212>  PRT
<213>  artificial

<220>
<223>  EpCAM peptide

<400>  3

Cys Gly Ser Lys Leu Gly Arg Arg Ala Lys Pro Glu Gly Ala Leu
1               5                   10                  15